# EUROPEAN PATENT SPECIFICATION

(11) **EP 1 687 057 B1**
(45) Date of publication and mention of the grant of the patent: **22.06.2011**
(21) Application number: 04784153.1
(22) Date of filing: 15.09.2004
(51) Int. Cl.: A61N 1/05

(54) **MEDICAL ELECTRICAL LEAD SYSTEM INCLUDING PRE-FORMED J-SHAPE STYLET**
MEDIZINISCHES ELEKTRISCHES LEITUNGSSYSTEM MIT VORGEFORMTEM J-FÖRMIGEM MANDRIN
SYSTEME DE CONDUCTEUR ELECTRIQUE MEDICAL COMPORTANT UN STYLET PREFORME EN FORME DE J

(30) Priority: 23.09.2003 US 668789
(43) Date of publication of application: 09.08.2006
(73) Proprietor: Medtronic, Inc., Minneapolis, MN 55432-5604 (US)
(72) Inventor: ZHAO, Yong, D., Plymouth, MN 55446 (US); HESS, Douglas, N., Maple Grove, MN 55311 (US); LENERS, Michael, R., East Bethel, MN 55011 (US)
(74) Representative: Hughes, Andrea Michelle
(86) International application number: PCT/US2004/030199
(87) International publication number: WO 2005/030319

(56) References cited:
- EP-A- 0 709 111
- EP-A- 0 715 865
- US-A1- 2002 049 485

## Description

The present invention relates generally to the field of implantable medical electrical stimulation and/or sensing leads, and particularly to pre-formed J-shape stylets used during transvenous placement of such leads.

Implantable medical electrical stimulation and/or sensing leads are well known in the fields of cardiac stimulation and monitoring, including cardiac pacing and cardioversion/defibrillation, and in other fields of electrical stimulation or monitoring of electrical signals or other physiologic parameters. An endocardial lead is typically placed through a transvenous route to locate one or more sensing and/or stimulation electrodes of the lead in a desired location of a chamber or vessel of the heart. Endocardial leads are typically advanced through an introducer lumen of an introducer extending from a skin incision into a vein, then through a venous pathway into the superior vena cava, and then into right atrium, right ventricle or coronary sinus or elsewhere depending on the chosen implantation site. A typical atrial implantation site is within the right atrial appendage and a typical ventricular pacing site is within the trabeculae of the right ventricular apex, while left atrial and ventricular implantation sites are accessed typically through the coronary sinus.

An elongated stylet wire including a proximal handle is often used to facilitate implantation of endocardial leads. The stylet wire distal end is inserted through a proximal connector pin opening, and the stylet wire is advanced through a lead lumen to impart rigidity or column strength to the lead, making it easier to advance the lead through the transvenous pathway. EP-A-0709111 and US 2002/049485 disclose such a stylet. Additionally, a stylet wire may be shaped in order to steer one or more electrodes of the lead to a particular implant site.

EP 0715865 teaches aj-shaped stylet, according to the preamble of claim 1. A pre-formed j-shape stylet is particularly useful in steering a lead to an implant site in the appendage of the right atrium. However, once the lead electrode is fixed to the implant site, the j-shape stylet must be removed from the lead without dislodging the electrode. Consequently, there is a need for pre-formed J-shape stylet that performs satisfactorily while avoiding dislodgement of the lead upon removal.

The present invention thus provides a stylet as claimed in claim 1 and a medical electrical lead system including such a stylet.

### BRIEF DESCRIPTION OF THE DRAWINGS

The following drawings are illustrative of particular embodiments of the invention and therefore do not limit its scope, but are presented to assist in providing a proper understanding of the invention. The drawings are not to scale (unless so stated) and are intended for use in conjunction with the explanations in the following detailed description. The present invention will hereinafter be described in conjunction with the appended drawings, wherein like numerals denote like elements, and:
FIG. 1 is a plan view with partial cut-away sections of an endocardial lead in which embodiments of the present invention may be implemented;
FIG. 2 is an enlarged section view of a distal end segment of the lead shown in FIG. 1;
FIG. 3 is a plan view of a distal portion of a pre-formed j-shape stylet wire according to one embodiment of the present invention; and
FIG. 4 is a plan view of a portion of a pre-formed j-shape stylet wire according to an alternate embodiment of the present invention.

### DETAILED DESCRIPTION

The following detailed description is exemplary in nature and provides a practical illustration for implementing exemplary embodiments of the invention. For convenience, stylets according to the present invention are described herein for implantation of an endocardial screw-in lead of the Bisping-type well known to those skilled in the art. Such endocardial screw-in leads include unipolar or bipolar screw-in pacing leads and cardioversion/defibrillation leads that support an elongated cardioversion/defibrillation electrode. However, it will be understood that the present invention may be practiced in conjunction with any type of endocardial lead.

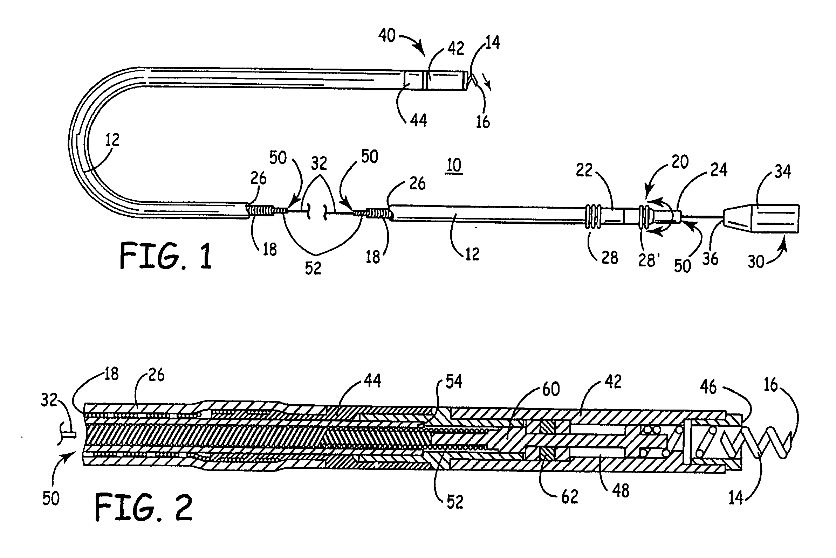

FIG. 1 is a plan view with partial cut-away sections of an endocardial lead in which embodiments of the present invention may be implemented; and FIG. 2 is an enlarged section view of a distal end segment of the lead shown in FIG. 1. FIG. 1 illustrates an endocardial screw-in lead 10 including an elongated lead body 12 extending between a connector assembly 20 and a distal electrode assembly or head 40; lead body 12 includes an outer insulating sheath 26, extending between connector assembly 20 and distal head 40, an elongated, single filar or multi-filar, outer coiled wire conductor 18 disposed within outer sheath 26, an elongated inner insulating sheath 54 (FIG. 2) extending within outer conductor 18 and an elongated, single filar or multi-filar, inner coiled wire conductor 52 disposed within inner sheath 54 and forming a portion of a stylet lumen 50. Insulating sheaths 26, 54 may be formed of any appropriate electrically insulative biocompatible and biostable material know those skilled in the art, for example silicone rubber or polyurethane, while conductors 18, 52 may be formed of any appropriate conductive material known in the art, for example MP35N alloy.

FIG. 1 further illustrates connector assembly 20 including a distal connector ring 22, a proximal connector pin 24 and proximal and distal sealing rings 28' and 28, respectively; connector pin 24 is formed of a metal tube having a tube lumen aligned with lumen of the lead body to form stylet lumen 50. A removable stylet 30 is shown in FIG. 1 including a proximal stylet knob 34 and an elongated stylet wire 32 inserted within lumen 50. Connector assembly 20 is adapted to be fitted into a bore of an IPG, once stylet 30 is removed, to make electrical connections between the connector ring 22 and pin 24 and IPG connector elements within the bore in a manner well known in the art.

Distal electrode head 40, shown in greater detail in FIG. 2, includes a proximal pace/sense ring electrode 44 and a distal tip fixation helix 14 functioning as a distal pace/sense electrode. FIGs. 1 and 2 further illustrate inner conductor coupling connector pin 24 of connector assembly 20 to a conductive helix driver 60 and outer conductor 18 coupling connector ring 22 of connector assembly 20 to ring electrode 44 positioned just proximal to head 40. Distal fixation helix 14 terminates in a sharpened point 16 for penetrating an implant site when fixation helix 14 is advanced past a distal end 46 from a chamber 48 within a housing 42 of electrode head 40 upon rotation of proximal connector pin 24 with respect to lead body 12. A proximal end of distal fixation helix 14 is affixed to a distal end of a helix driver 60 coupled to inner conductor 52 and supported within chamber 48 by a sealing ring assembly 62 and cylindrical chamber 48 is sufficient in length to receive fixation helix 14 retracted therein.

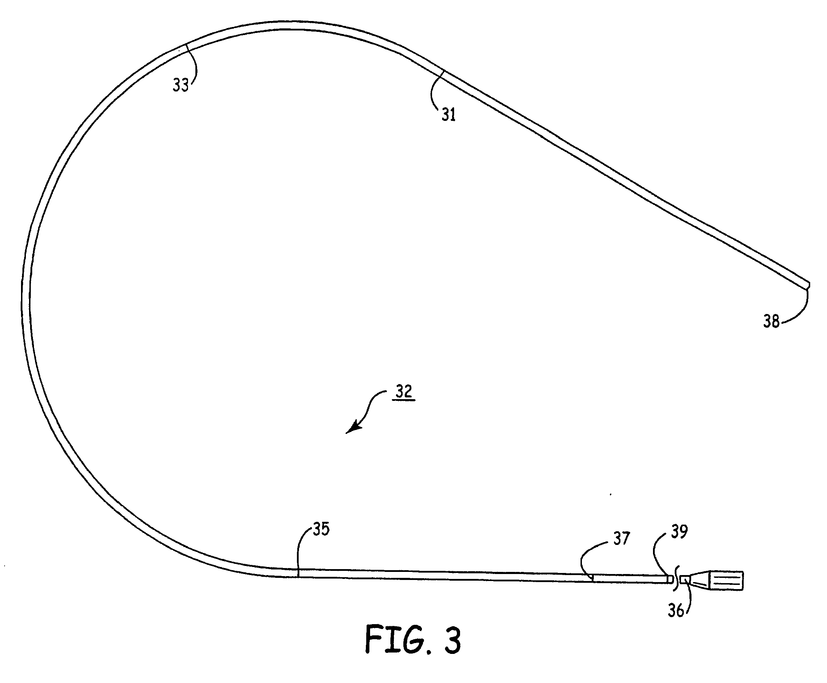

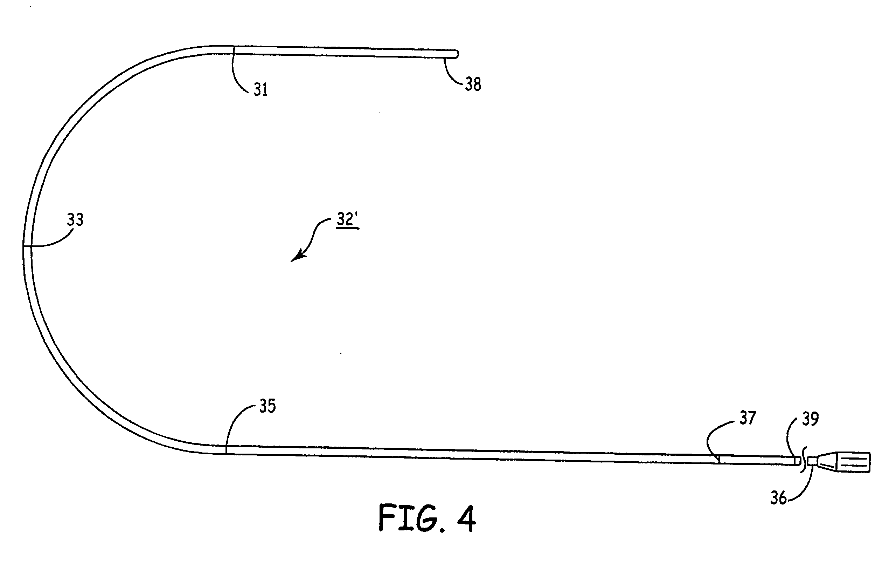

During implantation, a stylet wire according to the present invention is inserted into lumen 50 imparting a j-shape to a distal portion of lead 10 in order to direct head 40 to an implant site, for example in a right atrial appendage or elsewhere along another atrial wall. Helix 14 is then axially extended distally from distal end 46 to penetrate the endocardium in a manner well known in the art. Once lead 10 is fixed at the implant site by helix 14, it is necessary to retract the stylet wire from lumen 50 without dislodging lead 10 from the implant site. According to embodiments of the present invention, presented in FIGs. 3 and 4, retraction forces imparted to the lead body 12 are minimized to prevent such dislodgement.

FIG. 3 is a plan view of a distal portion of a j-shape stylet wire according to one embodiment of the present invention; and FIG. 4 is a plan view of a portion of a j-shape stylet wire according to an alternate embodiment of the present invention. FIGs. 3 and 4 illustrate two embodiments of stylet wires 32 and 32', respectively, including a substantially straight distal segment 38-31 (that is, extending from a distal end point 38 to a point 31), a curved intermediate segment 31-35, and a substantially straight proximal segment 35-36 (that is, extending from a point 35 to a proximal end point 36). FIG. 3 illustrates stylet wire 32 wherein curved intermediate segment 31-35 sweeps around approximately 210 degrees, while FIG. 4 illustrates stylet wire 32' wherein curved intermediate segment 31-35 sweeps around approximately 180 degrees. According to embodiments of the present invention radii of intermediate segments 31-35 are empirically selected based upon average right heart chamber sizes, so that, for example a radius for stylet wires 32, 32' is approximately 0.5 inch (1.27 cm).

FIGs. 3 and 4 further illustrate stylet wires 32, 32' including a proximal taper zone 37-39 (that is, extending between a point 37 and a point 39) located within proximal segment 35-36 and a distal taper zone 38-33 extending from distal end point 38 into curved intermediate segment 31-35. According to alternate embodiments of the present invention stylet wires 32, 32' include only proximal taper zone 37-39, wherein point 37 is located distal to point 35 either within the curved intermediate segment 31-35, according to one embodiment, or within substantially straight distal segment 38-31, according to another embodiment. According to another group of embodiments, stylet wires 32, 32' include only distal taper zone 38-33. Furthermore, it should be understood that point 33 might be positioned closer to point 35 than is illustrated in FIG. 3 and may even be coincident with point 35; likewise point 37 might be positioned closer to point 35 and may even be coincident with point 35. Additional features that may be included in embodiments of the present invention include a ball tip formed at distal end point 38, a specialized geometry of distal end point 38 adapted to interface with helix driver 60 as an alternative screw mechanism to retract and extend helix 14, and a lubricious coating formed on stylet wire.

### EXAMPLE

Explicit software packages (ProEngineer-SDRC-ABAQUS) were employed to mathematically model and compare two exemplary lead systems: a system #1 includes a lead similar to that described in conjunction with FIGs. 1 and 2 and pre-formed J-shape stylets according to the present invention; and a system #2 includes the same lead and a state-of-the art pre-formed J-shape stylet. Over 40,000 3-D triangular and brick elements with 3-D deformable to deformable contact between the lead and stylets were generated to form the non-linear finite element analysis models. The distal end of the lead was constrained, pin-pin, while continuously dragging stylets for approximately 1 inch from full insertion of the stylets within the lumen of the lead. The material properties loaded for the stylets were that of 304 stainless steel. Pullout reaction forces at a distal end of the leads, as the stylets were removed, were determined for each system; dimensions describing each system, with reference to the Figures, are presented in Table I and maximum pullout reaction forces are presented in Table 2.

**TABLE 1: Dimensions (in.) (1 in = 2.54 cm) describing stylets, reference FIGs. 3-4.**

| Zones/segments: | 38-31 | 38-33 | 33-37 | 35-39 | 37-39 |
|---|---|---|---|---|---|
| System will #1 stylets (double taper) | | | | | |
| Length | 0.85 (FIG. 3,210°) 0.55 (FIG. 4, 180°) | 1.38 (FIG. 3, 210°) 1.38 (FIG. 4, 180°) | 2.02 (FIG. 3,210°) 2.02 (FIG. 4, 180°) | 0.75 (FIG. 3, 210°) 1.46 (FIG. 4, 180°) | 0.20 (FIG. 3,210°) 0.20 (FIG. 4, 180°) |
| Diameter (same for 210° and 180°) | Within taper zone | Taper: 0.007 at 38 - 0.012 at 33 | 0.012 | 0.012 at 35 - 0.014 at 39 | Taper: 0.012 at 37 - 0.014 at 39 |
| System #2 stylets (single taper) | | | | | |
| Length | 0.85 (210°) 0.55 (180°) | 1.38 (210°) 1.38 (180°) | 2.02 (210°) 2.02 (180°) | 0.75 (210°) 1.46 (180°) | 0.20 (210°) 0.20 (180°) |
| Diameter (same for 210° and 180°) | Taper: 0.007 at 38 - 0.014 at 31 | 0.007 at 38 - 0.014 at 33 | 0.014 | 0.014 | 0.014 |

| | | | | | |
|---|---|---|---|---|---|
| Notes: Each stylet included a curve radius of 0.51 inch (1.3 cm). Each lead included a stylet lumen diameter of 0.017" (0.4318 mm). | | | | | |

**TABLE 2: Maximum pullout reaction forces (1b) (11b = 0.4536 kg). Normalized values are shown in brackets.**

| | 180° Curved stylet | 210° Curved stylet |
|---|---|---|
| System #1 (double tapered stylets) | 0.465 | 0.335 |
| | [1.4] | [1.0] |
| System #2 (single tapered stylets) | 3.051 | 5.417 |
| | [9] | [16] |

The above results indicate that stylets according to embodiments of the present invention produce significantly lower pullout reaction forces than state-of-the-art stylets and thus can reduce a probability of lead tip dislodgement upon stylet removal.

It will be understood that certain of the above-described structures, functions and operations of the above-described embodiments are not necessary to practice the present invention and are included in the description simply for completeness of an exemplary embodiments. For example, although the stylets modeled and presented in the EXAMPLE herein included two taper zones, a single taper zone, for example, either extending from a distal straight segment into a curved segment or extending from within the curved segment into a proximal straight segment, fall within the scope of the present invention.

## Claims

1. A pre-formed J-shape stylet for use with a medical electrical lead, comprising a stylet wire (32) adapted to be inserted into a lumen of the lead, said stylet wire having a distal portion comprising:
a substantially straight distal segment (38-31) extending from a distal end (38);
a curved intermediate segment (31-35) extending from the substantially straight distal segment; and
a substantially straight proximal segment (35-36) extending from the curved intermediate segment toward a proximal end; and **characterized by**
a taper zone (37-39; 38-33) extending within the curved intermediate segment.

2. The stylet of claim 1, wherein the taper zone extends from a first diameter within the substantially straight distal segment to a second diameter within the curved intermediate segment, the second diameter being greater than the first diameter.

3. The stylet of claim 2, wherein the first diameter within the substantially straight distal segment coincides with the distal end of the stylet.

4. The stylet of claim 1, wherein the taper zone extends from a first diameter within the substantially straight proximal segment to a second diameter within the curved intermediate segment, the first diameter being greater than the second diameter.

5. The stylet of any preceding claim, wherein the curved intermediate segment sweeps around 210 degrees.

6. The stylet of any of claims 1 to 4, wherein the curved intermediate segment sweeps around 180 degrees.

7. The stylet of any of claims 1 to 4 wherein the curved intermediate segment sweeps around between 180 and 210 degrees.

8. The stylet of claim 2 or any claim dependent thereon, further comprising a second taper zone extending distally from a third diameter within the substantially straight proximal segment to a fourth diameter, the third diameter being greater than the fourth diameter and the fourth diameter being approximately equal to the second diameter.

9. The stylet of claim 8, wherein the first diameter within the substantially straight distal segment coincides with the distal end of the stylet.

10. The stylet of claim 8, wherein the fourth diameter resides within the substantially straight proximal segment.

11. The stylet of claim 8, wherein the fourth diameter resides within the intermediate segment.

12. A medical electrical lead system, comprising
a medical electrical lead (10) including a proximal end, a distal portion, and an elongated lumen (50) extending from the proximal end into the distal portion; and
a pre-formed J-shape stylet (32) as claimed in any preceding claim;
wherein the j-shape stylet is slidably received within the lumen of the lead such that the curved intermediate segment of the stylet imparts a similar curve to the distal portion of the lead.

13. The medical electrical lead system of claim 12, wherein the lead further includes an extendable/retractable helix (14) terminating the distal portion of the lead.

## Patentansprüche

1. Vorgeformte, J-förmige Lanzette zur Verwendung mit einer medizinischen elektrischen Leitung, mit einem Lanzettendraht (32), der dafür eingerichtet ist, in ein Lumen der Leitung eingesetzt zu werden, wobei der Lanzettendraht einen Distalabschnitt besitzt, der aufweist:
ein im Wesentlichen gerades Distalsegment (28-31), das sich von einem Distalende (38) erstreckt;
ein gekrümmtes Zwischensegment (31-35) das sich von dem im Wesentlichen geraden Distalsegment erstreckt;
ein im Wesentlichen gerades Poximalsegment (35-36), das sich von dem gekrümmten Zwischensegment zu in einem Proximalende erstreckt; und **gekennzeichnet durch**
eine Verjüngungszone (37-39; 38-33), die sich innerhalb des gekrümmten Zwischensegments erstreckt.

2. Lanzette nach Anspruch 1, bei der die Verjüngungszone sich von einem ersten Durchmesser innerhalb des im Wesentlichen geraden Distalsegments zu einem zweiten Durchmesser innerhalb des gekrümmten Zwischensegments erstreckt, wobei der zweite Durchmesser größer als der erste Durchmesser ist.

3. Lanzette nach Anspruch 2, bei der der erste Durchmesser innerhalb des im Wesentlichen geraden Distalsegments mit dem Distalende der Lanzette übereinstimmt bzw. zusammenfällt.

4. Lanzette nach Anspruch 1, bei der die Verjüngungszone sich von einem ersten Durchmesser innerhalb des im Wesentlichen geraden Proximalsegments zu einem zweiten Durchmesser innerhalb des gekrümmten Zwischensegments erstreckt, wobei der erste Durchmesser größer als der zweite Durchmesser ist.

5. Lanzette nach einem der vorstehenden Ansprüche, bei der das gekrümmte Zwischensegment 210 Grad überstreicht bzw. umfasst,

6. Lanzette nach einem der Ansprüche 1 bis 4, bei der das gekrümmte Zwischensegment 180 Grad überstreicht.

7. Lanzette nach einem der Ansprüche 1 bis 4, bei der das gekrümmte Zwischensegment zwischen 180 Grad und 210 Grad überstreicht.

8. Lanzette nach Anspruch 2 oder einem hiervon abhängenden Anspruch, ferner mit einer zweiten Verjüngungszone, die sich distal von einem dritten Durchmesser innerhalb des im Wesentlichen geraden Proximalsegments zu einem vierten Durchmesser erstreckt, wobei der dritte Durchmesser größer als der vierte Durchmesser und der vierte Durchmesser im Wesentlichen gleich dem zweiten Durchmesser ist.

9. Lanzette nach Anspruch 8, bei der der erste Durchmesser innerhalb des im Wesentlichen geraden Distalsegments mit dem Distalende der Lanzette zusammenfällt.

10. Lanzette nach Anspruch 8, bei der der vierte Durchmesser in dem im Wesentlichen geraden Proximalsegment sitzt.

11. Lanzette nach Anspruch 8, bei der der vierte Durchmesser innerhalb des Zwischensegments sitzt.

12. Medizinisches elektrisches Leitungssystem mit
einer medizinischen elektrischen Leitung (10) mit einem Proximalende, einem Distalabschnitt und einem länglichen Lumen (50), das sich von dem Proximalende in den Distalabschnitt erstreckt; und
einer vorgeformten J-förmigen Lanzette (32) nach einem der vorstehenden Ansprüche;
Wobei die j-förmige Lanzette gleitend in dem Lumen der Leitung aufgenommen ist, so dass das gekrümmte Zwischensegment der Lanzette eine ähnliche Krümmung auf den Distalabschnitt der Leitung aufbringt.

13. Medizinisches elektrisches Leitungssystem nach Anspruch 12, bei dem die Leitung ferner eine erstreckbare/zurückziehbare Schnecke (14) aufweist, die den Distalabschnitt der Leitung abschließt.

## Revendications

1. Stylet en forme de J préformé à utiliser avec une dérivation électrique médicale, contenant un fil de stylet (32) adapté en vue d'une insertion dans une lumière de la dérivation, ledit stylet ayant une partie distale comportant :
un segment distal sensiblement droit (38-31) s'étendant depuis l'extrémité distale (38) ;
un segment intermédiaire incurvé (31-35) s'étendant depuis le segment distal sensiblement droit ; et
un segment proximal sensiblement droit (35-36) s'étendant depuis le segment intermédiaire incurvé vers une extrémité proximale ; et **caractérisé par**
une zone effilée (37-39 ; 38-33) s'étendant dans le segment intermédiaire incurvé.

2. Stylet selon la revendication 1, dans lequel la zone effilée s'étend depuis un premier diamètre dans le segment distal sensiblement droit jusqu'à un second diamètre dans le segment intermédiaire incurvé, le second diamètre étant supérieur au premier diamètre.

3. Stylet selon la revendication 2, dans lequel le premier diamètre dans le segment distal sensiblement droit coïncide avec l'extrémité distale du stylet.

4. Stylet selon la revendication 1, dans lequel la zone effilée s'étend depuis un premier diamètre dans le segment proximal sensiblement droit jusqu'à un second diamètre dans le segment intermédiaire incurvé, le premier diamètre étant supérieur au second diamètre.

5. Stylet selon l'une quelconque des revendications précédentes, dans lequel le segment intermédiaire incurvé balaie environ 210 degrés.

6. Stylet selon l'une quelconque des revendications 1 à 4, dans lequel le segment intermédiaire incurvé balaie environ 180 degrés.

7. Stylet selon l'une quelconque des revendications 1 à 4, dans lequel le segment intermédiaire incurvé balaie environ entre 180 et 210 degrés.

8. Stylet selon la revendication 2 ou l'une quelconque des revendications dépendantes, comportant en outre une seconde zone effilée s'étendant de manière distale depuis un troisième diamètre dans le segment proximal sensiblement droit jusqu'à un quatrième diamètre, le troisième diamètre étant supérieur au quatrième diamètre et le quatrième diamètre étant approximativement égal au deuxième diamètre.

9. Stylet selon la revendication 8, dans lequel le premier diamètre dans le segment distal sensiblement droit coïncide avec l'extrémité distale du stylet.

10. Stylet selon la revendication 8, dans lequel le quatrième diamètre réside dans le segment proximal sensiblement droit.

11. Stylet selon la revendication 8, dans lequel le quatrième diamètre réside dans le segment intermédiaire.

12. Système de dérivation électrique médicale, comportant :
une dérivation électrique médicale (10) incluant une extrémité proximale, une partie distale, et une lumière allongée (50) s'étendant depuis l'extrémité proximale dans la partie distale ; et
un stylet en forme de J préformé (32) comme revendiqué dans l'une quelconque des revendications précédentes ;
dans lequel le stylet en forme de J est reçu par coulissement dans la lumière de la dérivation de sorte que le segment intermédiaire incurvé du stylet imprime une courbe similaire à la partie distale de la dérivation.

13. Système de dérivation électrique médicale selon la revendication 12, dans lequel la dérivation inclut en outre une hélice extensible/rétractable (14) terminant la partie distale de la dérivation.
